# FASCICULE DE BREVET EUROPEEN

(11) **EP 0 454 509 B1**
(45) Date de publication et mention de la délivrance du brevet: **26.06.1996**
(21) Numéro de dépôt: 91400722.4
(22) Date de dépôt: 18.03.1991
(51) Int. Cl.: G01N 33/80

(54) **Procédé de mise en évidence d'agglutination érythrocytaire destiné aux analyses de compatibilités sanguines**
Verfahren zum Nachweis der Erythrozytenagglutination zur Feststellung der Blutverträglichkeit
Method to reveal erythrocyte agglutination for the determination of blood compatybility

(30) Priorité: 27.03.1990 FR 9003873
(43) Date de publication de la demande: 30.10.1991
(73) Titulaire: CENTRE REGIONAL DE TRANSFUSION SANGUINE DE LILLE, F-59012 Lille (FR)
(72) Inventeur: Mannessier, Lucienne née Wartelle, F-59000 Lille (FR)
(74) Mandataire: Lhuillier, René

(56) Documents cités:
- EP-A- 0 039 195
- EP-A- 0 194 212
- EP-A- 0 305 337
- GB-A- 2 117 514
- RESEARCH DISCLOSURE, vol. 200, décembre 1980, Havant (GB); p. 558/

## Description

L'invention concerne un procédé de mise en évidence d'agglutination érythrocytaire utilisé en immunohématologie transfusionnelle, de suivi de grossesse et de diagnostic, qui apporte une augmentation significative de sensibilité et de fiabilité par rapport aux autres méthodes connues.

La diminution de la fréquence des accidents de transfusion sanguine et d'incompatibilité foeto-maternelle est le résultat du grand nombre de tests de compatibilité mis en oeuvre et de l'amélioration constante de leur spécificité et de leur fiabilité.

Ces tests dont le nombre et les modalités d'exécution sont fixés par la loi comprennent le groupage ABO et Rhésus standard, la recherche des anticorps irréguliers antiérythrocytaires, les tests de compatibilité et la détermination fine des phénotypes érythrocytaires.
- Les tests de groupage sanguin ABO-Rh standard comprennent une "épreuve globulaire" de détection des antigènes à la surface des érythrocytes (ou hématies ou globules rouges) avec des antisérums standardisés et, pour le groupe ABO, une "épreuve sérique" de détection des anticorps présents dans le sang et dressés contre les antigènes érythrocytaires à l'aide d'érythrocytes-tests.
- La recherche des anticorps irréguliers, c'est-à-dire des anticorps autres que anti-A et -B, et qui ne sont pas communs à l'ensemble de la population, constitue la base de la sécurité immunologique des transfusions et de la prévention des accidents d'allo-immunisation foeto-maternelle.
- La détermination précise des phénotypes érythrocytaires du donneur et du receveur est nécessaire quand on a détecté des anticorps irréguliers chez le receveur ; elle est recommandée chez les patients nécessitant des transfusions itératives ; elle est obligatoire chez la femme enceinte. En effet, en cas de besoin transfusionnel, le malade doit impérativement être transfusé avec du sang phénotypé (sang du donneur) c'est-à-dire dépourvu de l'antigène correspondant à l'anticorps identifié, et compatibilisé au laboratoire.

Ces différents tests sont réalisés par des techniques classiques d'immunohématologie qui comprennent une mise en évidence d'agglutination érythrocytaire (due à la réaction antigène-anticorps), - soit directe, en milieu salin, éventuellement après traitement des érythrocytes avec une enzyme (trypsine, papaïne, broméline) dont l'action sur les membranes diminue la répulsion spontanée des érythrocytes (due aux charges négatives des groupements carboxyliques des molécules d'acide sialique présentes à la surface des érythrocytes),
- soit indirecte, c'est-à-dire en deux étapes, d'abord une sensibilisation des érythrocytes avec les anticorps (qui ne sont pas nécessairement agglutinants), puis leur agglutination par addition d'antiglobuline polyvalente. Cette réaction est effectuée en présence d'anti-complément. Cette technique connue sous le nom de "Coombs indirect" est particulièrement longue et délicate et laisse plusieurs possibilités d'erreurs de manipulation et de lecture du résultat.

Le domaine d'application de ces tests comprend la recherche d'agglutinines irrégulières, les épreuves de compatibilité pré-transfusionnelle et foeto-maternelle, différents diagnostics d'accidents hémolytiques (maladie hémolytique du nouveau-né, anémie hémolytique auto-immune, hémolyse post-transfusionnelle...).

Le très grand nombre de tests actuellement pratiqués et légalement exigés tant pour la sécurité pré-transfusionnelle que pour le suivi des grossesses rend très souhaitable la recherche de perfectionnements techniques pour faciliter la réalisation et la lecture du test tout en augmentant la fiabilité du résultat.

Un progrès significatif a déjà été apporté par l'utilisation d'un milieu gélifié et d'une étape de centrifugation douce pour séparer les globules du sérum (Mollison - Blood transfusion in clinical medicine - éd. Blackwell - Oxford - 1983 - p.512).

Un progrès supplémentaire a été réalisé par l'application du même principe à la rétention sélective d'agglutinats érythrocytaires, par analogie avec une technique classique en immunologie pour mettre en évidence, en gel, les réseaux agglutinés d'antigènes-anticorps.

Ce procédé, décrit dans le brevet EP-A-0 194.212, consiste en une centrifugation douce des échantillons à tester dans un tube à fond conique rempli de gel de dextran ; il permet une lecture facile même par un technicien peu expérimenté, parce que les érythrocytes non agglutinés (test négatif) sédimentent très nettement au fond du tube alors que les agglutinats d'érythrocytes liés par les anticorps sont ralentis et retenus dans le gel et ceci d'autant plus que la réponse est fortement positive. Ce type de test est appelé en abrégé "gel-centrifugation".

Le procédé breveté a été développé et commercialisé sous forme de "kits" par la société Dia Med (SA - Morat CH 3280 - Suisse ou 75002 Paris France). Ces "kits" permettent d'effectuer un grand nombre de tests en peu de temps et sont faciles à lire. Toutefois ils n'offrent pas la même sécurité que les tests classiques en tubes et on constate des discordances significatives dans la détection de certaines agglutinines irrégulières qui peuvent être responsables d'accidents transfusionnels.

La Demanderesse a donc cherché à augmenter la précision de ce type de test en "gel-centrifugation" pour garantir la fiabilité du résultat.

Plus particulièrement, la Demanderesse a remarqué qu'on pouvait augmenter le pouvoir de discrimination du gel par l'incorporation d'un deuxième gel, de réticulation plus fine. Ainsi on mélangera avantageusement du Sephadex^{®} G100 (dont les particules ont un diamètre de 20 à 50 µm) et du Sephasorb^{®} HP Ultrafine(dont les particules ont un diamètre de 10 à 23 µm) ; ces deux produits sont des dextrans commercialisés par Pharmacia (Pharmacia Fine Chemicals - Uppsala - Suède) . D'autres matériaux similaires et classiquement utilisés pour les séparations chromatographiques par simple tamisage moléculaire peuvent également être utilisés.

Différents réactifs spécifiques de chaque type de test sont avantageusement incorporés dans le gel.
Ainsi, - pour le test en milieu salin et pour le test après traitement des hématies aux enzymes, on incorpore un sérum AB humain préalablement contrôlé, ou des immunoglobulines purifiées, afin de faciliter la sédimentation des globules non agglutinés ;
- pour les tests d'agglutination directe ou indirecte de Coombs (ou tout autre test dérivé de ceux-ci), on incorpore les antiglobulines polyvalentes ou spécifiques et l'anti-complément dans le gel.

. Les antiglobulines entraînent une agglutination des hématies sensibilisées par un processus immunologique.
. Les antiglobulines humaines sont de nature IgG et jouent le même rôle que le sérum AB humain ou les IgG humaines purifiées, à savoir d'améliorer la sédimentation des hématies non agglutinées.

Comme dans les tests classiques, les antiglobulines utilisées pour l'agglutination indirecte sont des antiglobulines polyvalentes et/ou des anti-IgG polyclonales de lapin et/ou des anti-compléments de lapin.

La Demanderesse a très fortement amélioré les performances du test de Coombs direct et indirect en remplaçant l'anti-complément standard par un anticorps monoclonal de spécificité anti-C₃d, dont elle a sélectionné l'hybridome suivant des critères choisis en vue de ce test.

Les conditions de mise en oeuvre de chaque test ont été particulièrement adaptées à cette technique de "gel-centrifugation". Ainsi la Demanderesse a constaté que la qualité de la réaction du test de Coombs en tampon à basse force ionique pouvait être augmentée par la qualité de la préparation d'érythrocytes (qui sont classiquement en suspension dans une solution saline à basse force ionique, comme décrit par Löw et Messeter - Vox Sang. 26, 1974, 53-61) et, en particulier, par l'addition dans cette suspension de 0,26 % d'EDTA disodique, choisi pour son rôle de chélateur du calcium ionisé ; il exerce ainsi un effet protecteur contre l'hémolyse des hématies (le milieu de basse force ionique provoquant une sensibilisation "non spécifique" des hématies par des fractions du complément présentes dans les sérums à tester). Il faut noter que à trop forte concentration d'EDTA, les anticorps fixant le complément ne sont plus détectés. Cet effet peut être compensé par le "dopage" de l'antiglobuline polyvalente avec l'anti-C₃d monoclonal purifié et concentré afin de mieux détecter les anticorps fixant le complément tels les anti-Jka sans pour autant amoindrir l'activité anti-IgG.

La Demanderesse a également augmenté les performances du test par le choix d'anticorps standards particulièrement performants dont plusieurs sont des anticorps monoclonaux.

Dans un cas spécifique et particulièrement difficile à identifier, comme le phénotypage Fya, la Demanderesse a montré que l'addition de méthylcellulose dans la solution d'anticorps améliorait la sensibilité de la réaction.

La présente invention concerne donc un procédé de mise en évidence d'agglutinats érythrocytaires caractérisé par un ensemble d'opérations toutes adaptées particulièrement au procédé en "gel-centrifugation". Plus particulièrement l'invention concerne :
- l'utilisation comme gel d'un mélange de 2 dextrans de finesse de réticulation différente ;
- l'addition dans ce gel des réactifs adaptés au type de test à réaliser, c'est-à-dire du sérum AB (ou des immunoglobulines purifiées) ou du mélange antiglobuline (polyvalente ou spécifique) - anti-complément ;
- l'utilisation d'un milieu contenant de l'EDTA disodique pour mettre en suspension les hématies qui seront utilisées dans le test ce qui augmentera le pouvoir de discrimination du test en préservant les hématies de l'hémolyse.

L'utilisation d'un anticorps monoclonal de spécificité anti-C₃d à forte affinité et très haut titre au lieu d'un anticorps anti-complément standard, ainsi que la sélection de l'hybridome produisant cet anticorps monoclonal font également partie de l'invention. En particulier, le criblage des hydridomes a été effectué en titrage, pour favoriser la sélection d'une lignée productrice d'anticorps monoclonal de très haut titre, c'est-à-dire encore positif en dilution 1/512.

Les exemples suivants illustrent l'invention sans en limiter la portée.

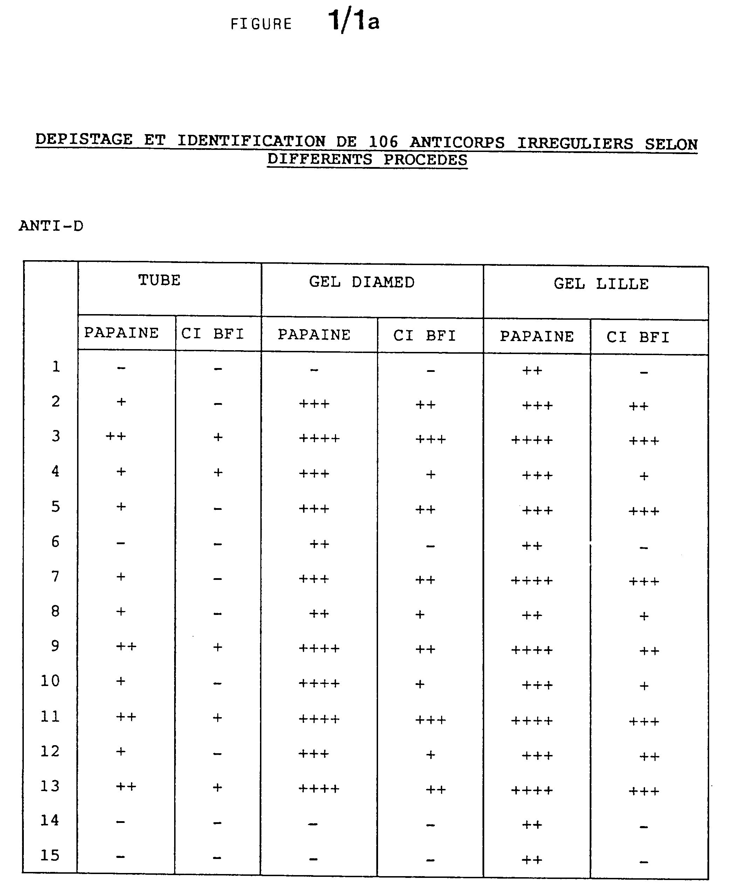

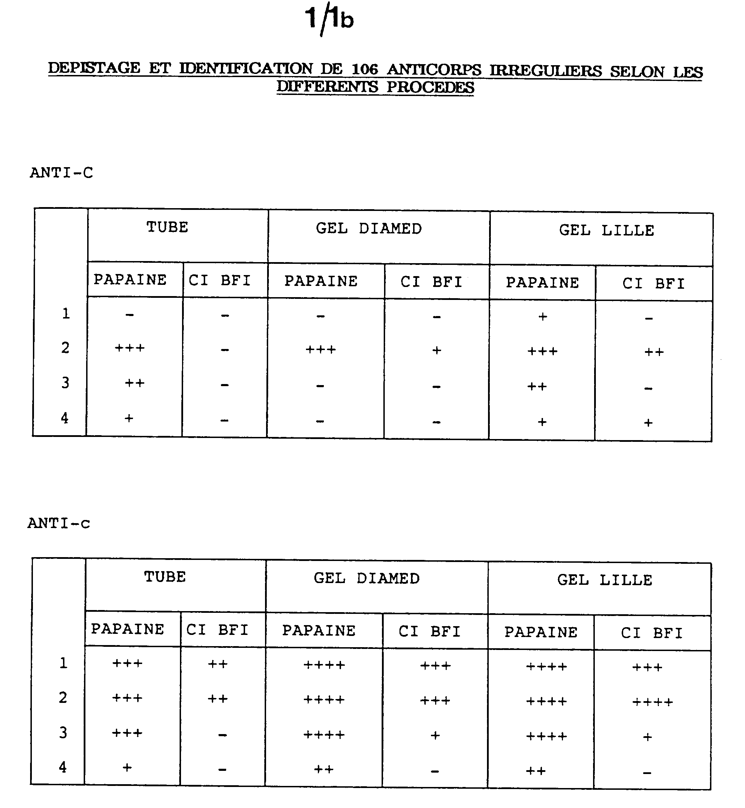

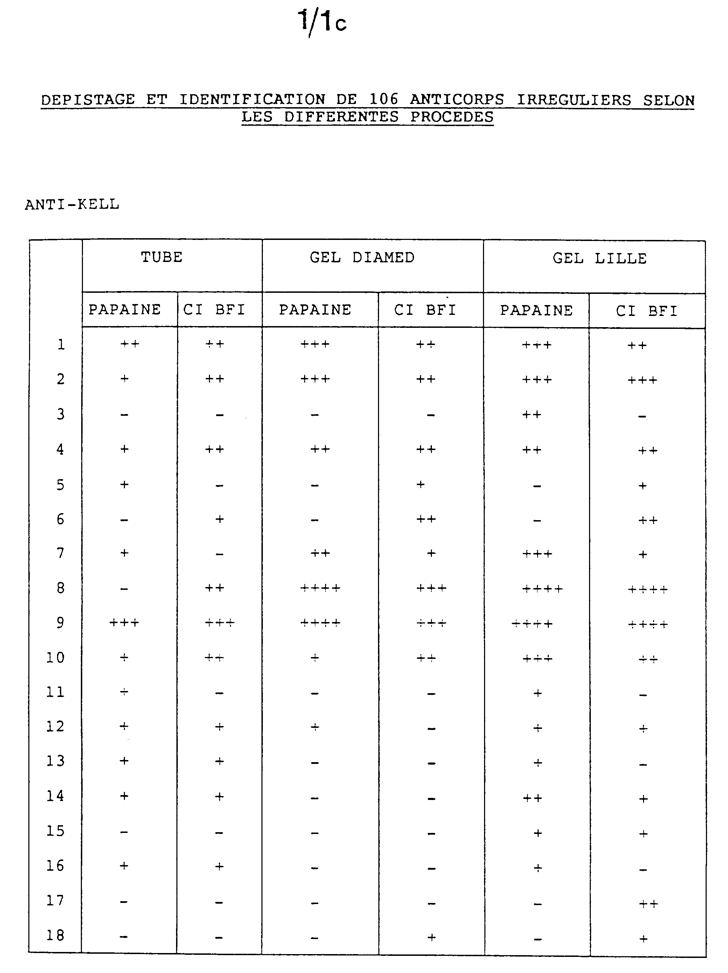

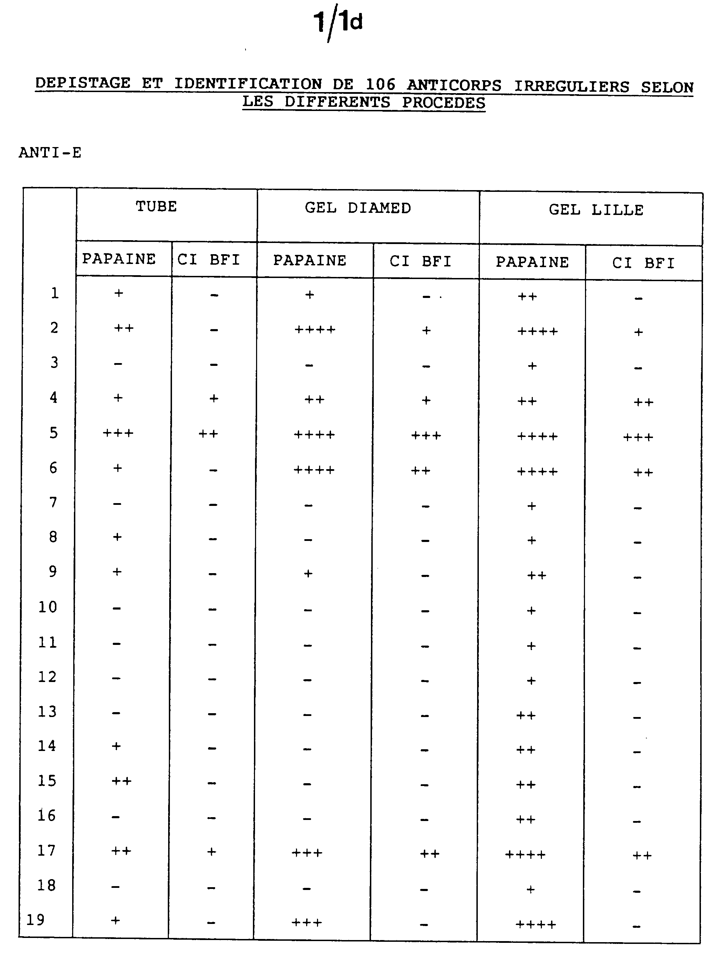

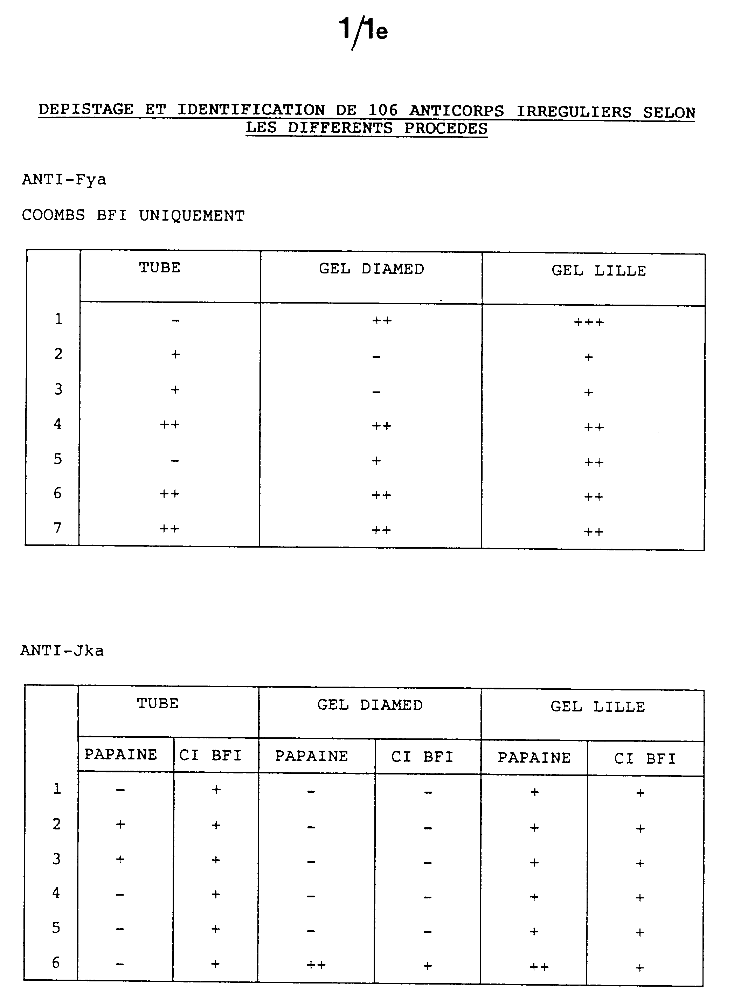

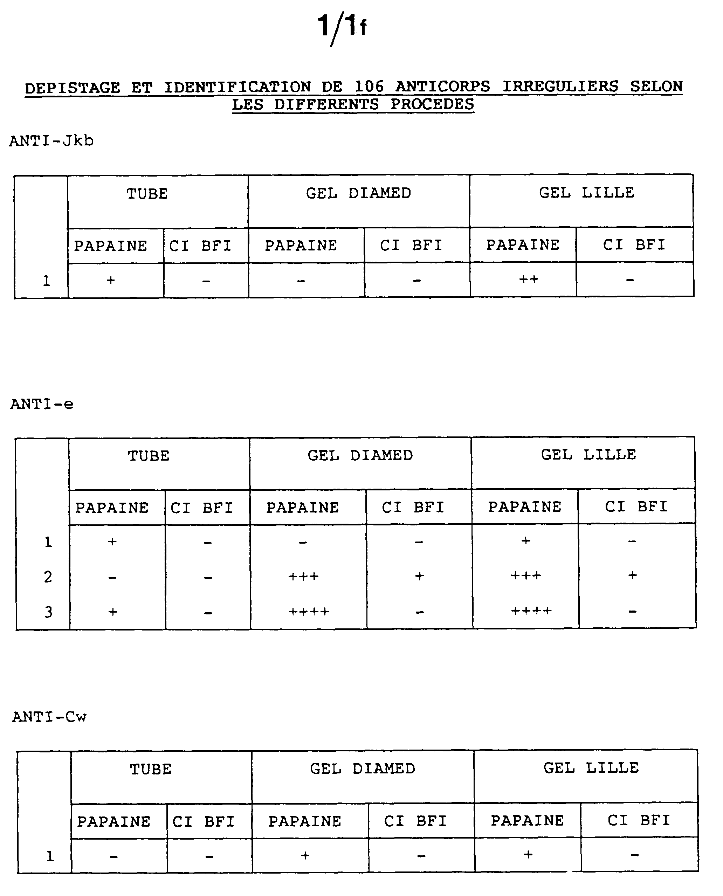

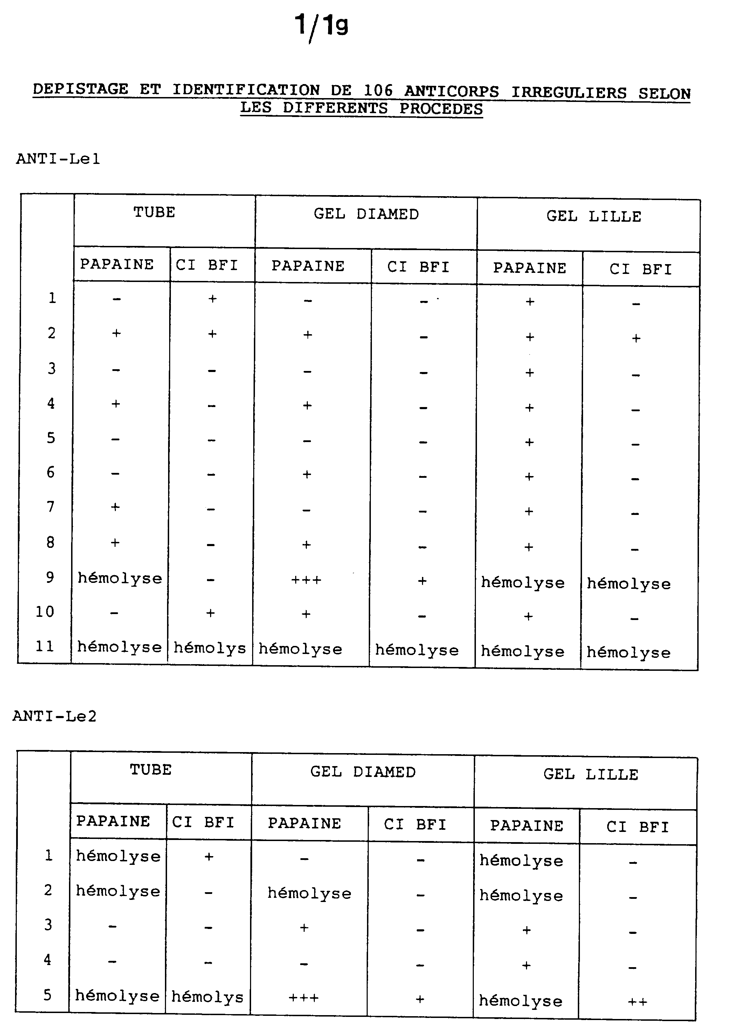

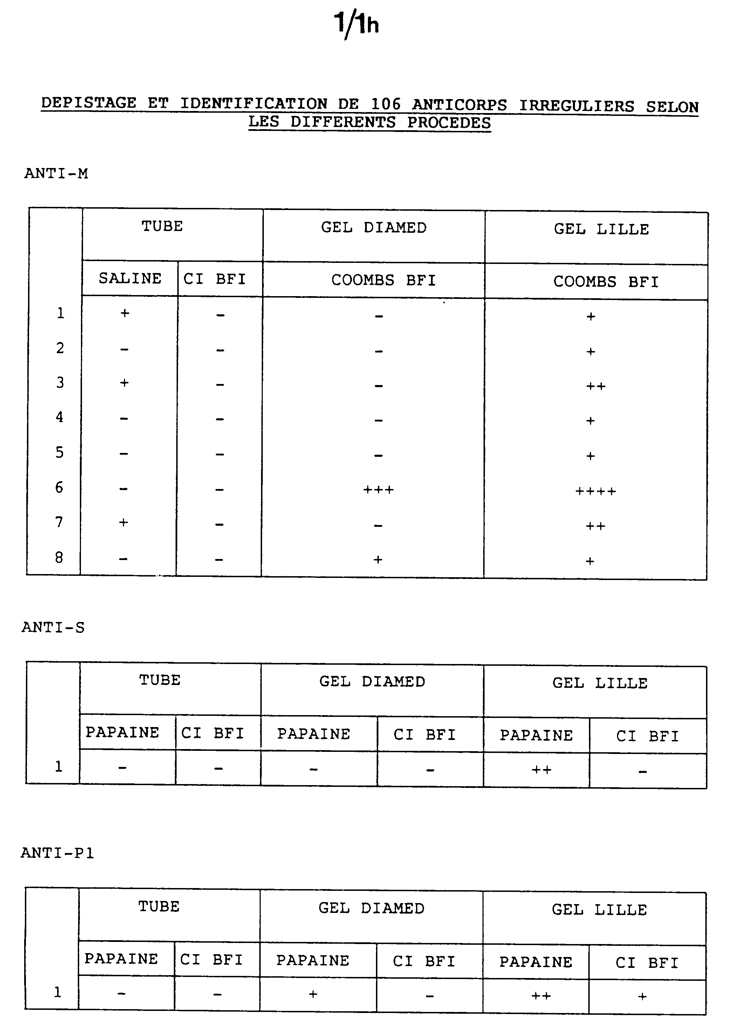

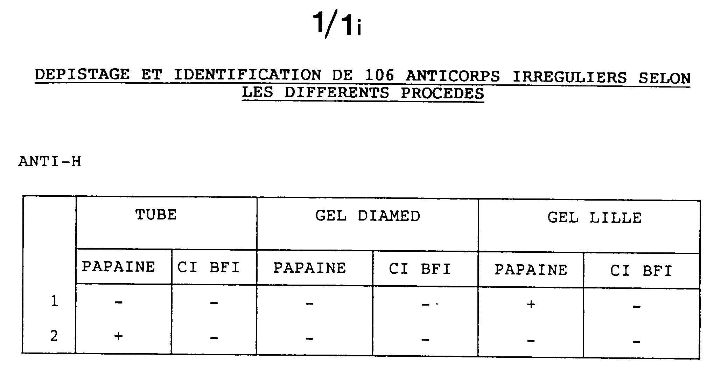

La figure 1 illustre un exemple d'application de l'invention sous forme de tableau des résultats comparés du dépistage et de l'identification de 106 anticorps irréguliers, par 3 procédés : le procédé de référence en tube (noté "tube"), le procédé vendu sous forme de "kit" par Dia Med (noté "gel Dia Med") et le procédé selon la présente invention (noté "gel Lille") ; 2 types de tests sont effectués en parallèle : le test enzymatique (noté "papaïne") et le test de Coombs indirect en milieu à basse force ionique (noté CI-BFI).

### EXEMPLE I.- Préparation des gels et répartition en microtubes.

### I.a Composition du gel de base commun à tous les tests.

Le gel est constitué de 2 dextrans de finesse de réticulation différente :
- le Sephadex^{®} G100 Superfine (20 - 50 µm)
- le Sephasorb^{®} HP Ultrafine (10 - 23 µm).

Ces 2 produits sont commercialisés par Pharmacia Fine Chemicals (Uppsala - Suède).

Les proportions des 2 substances varient en fonction du test à effectuer :
- pour les tests de Coombs on utilise
   5 g de Sephadex^{®} et 5 mg (ou 10 mg) de Sephasorb
- pour les tests en milieu salin et les tests enzymatiques on utilise
   5 g de Sephadex^{®} et 5 mg (ou 10 mg) de Sephasorb.

Ce mélange est mis en suspension dans 90 ml (ou 100 ml) de "solution saline à basse force ionique" (milieu classique mis au point par Löw et Messeter - Vox Sang. 26, 1974, 53-61) qui contient du NaCl 0,03 M.

### I.b. Réactifs spécifiques

Pour le test de Coombs à l'antiglobuline, on incorpore dans le gel 15 ml d'antiglobuline polyvalente (anti-IgG polyclonale de lapin) et d'anti-complément (anti-C₃d monoclonal de souris - voir plus loin -).

Pour le test enzymatique (c'est-à-dire quand on utilise des hématies préalablement traitées aux enzymes), on incorpore dans le gel 10 ml de sérum humain AB préalablement contrôlé (ou 100 mg d'immunoglobulines purifiées), ou éventuellement des immunoglobulines purifiées.

### I.c. Répartition du gel dans les microtubes

On utilise des microtubes à centrifuger, à fond conique (par exemple de type Elkay d'une contenance de 250 µl). Le gel est réparti à la pipette automatique à raison de 100 µl par tube.

Les tubes sont centrifugés 2 minutes à 1500 g pour tasser le gel et éliminer les bulles.

### EXEMPLE II. Mise en oeuvre des tests

### II.a. Préparation des hématies

Les prélèvements à étudier sont centrifugés 15 minutes à 2000 g.

Pour le test de Coombs, les hématies sont mises en suspension à 0,8 % (ou à 1 % pour certains tests) dans la "solution saline à basse force ionique" (voir plus haut), additionnée de 0,26 % d'EDTA disodique. Cette modification du milieu apporte une amélioration significative de la sédimentation des hématies au travers du gel quand la réaction est négative (sérum ne contenant pas d'anticorps dirigés contre les antigènes des hématies).

Pour les tests en milieu salin et les tests enzymatiques (papaïne, trypsine, broméline, effectués comme dans les méthodes classiques), les hématies sont mises en suspension à 0,8 % dans de l'eau physiologique (ou à 1 % pour certains tests).

### II.b. Mise en oeuvre du test

Dans chaque microtube on dépose à la surface du gel - dans un premier temps, 50 µl d'hématies en suspension (comme défini en IIa)
- dans un deuxième temps, 25 µl de sérum.
Après une incubation de 15 minutes au bain-Marie à 37°C les microtubes sont centrifugés pendant 9 minutes à 430 g et la lecture est effectuée macroscopiquement.
- Quant le test est négatif, les hématies forment un culot net au fond du tube.
- Quand le test est positif, les hématies agglutinées sont plus au moins retardées dans le gel ; les résultats sont notés par un nombre de + variant de 1 à 4.
- On peut observer une hémolyse des hématies traitées par les enzymes quand les anticorps fixent le complément (surnageant rosé).

### EXEMPLE III. Sélection d'un anticorps monoclonal anti-complément de spécificité anti-C₃d, à forte affinité et de très haut titre.

La méthode classique d'induction d'hybridomes de Kholer et Milstein par fusion de splénocytes de souris immunisées et de cellules de myélome murin non sécréteur d'immunoglobulines a été adaptée en vue de la préparation d'hybridomes sécréteurs d'anti-complément de spécificité anti-C₃d.

### 1) Choix de l'immunogène

Des souris sont immunisées avec des hématies de donneur sain, contrôlées, recouvertes de la fraction C₃d du complément. La préparation de ces hématies-C₃d est réalisée in vitro de la manière suivante :

### 1-a- Préparation d'hématies-C₃ par la méthode à basse force ionique à froid

- Préparer la solution A suivante :

| | |
|---|---|
| 1,0 M K₂HPO₄ | 1,25 ml |
| 0,2 M Na₂ EDTA | 5,25 ml |
| saccharose | 23,1 g |
| eau distillée QSP | 250 ml |

- Préparer la solution B suivante :

| | |
|---|---|
| 1,0 M KH₂PO₄ | 1,1 ml |
| 0,2 M Na₂ EDTA | 5,25 ml |
| saccharose | 23,1 g |
| eau distillée QSP | 250 ml |

- Préparer la solution C suivante :

| | |
|---|---|
| 2,0 M MgCl₂ 6H₂O | 10 ml |
| eau distillée QSP | 50 ml |

Préparer du tampon phosphate/saccharose pH 5,1 en ajoutant la solution A à la solution B jusqu'à obtention du pH désiré.
- Placer 19 ml de tampon phosphate/saccharose pH 5,1 dans un bécher de 50 ml plongé dans un bain de glace fondante. Mélanger jusqu'à ce que la température du tampon soit comprise entre 0 et 1°C,
- Ajouter 0,5 ml de culot d'hématies de groupe O, lavées trois fois.
- Ajouter 0,5 ml de sérum ou de plasma frais dilué à 1/50 en solution saline isotonique et 0,1 ml de solution C.
- Incuber trente minutes à 0°C.
- Centrifuger, éliminer le surnageant, laver quatre fois les hématies avec 20 ml de tampon PBS puis jeter le dernier surnageant de lavage.
- Ajouter 14,5 ml de tampon PBS au culot cellulaire. On obtient ainsi une suspension à 3 % d'hématies présentant le déterminant C₃b du complément à leur surface (ces hématies sont appelées EC₃b)

### 1-b- Préparation d'hématies-C₃d par traitement à la trypsine des hématies EC₃b.

- Diluer 2,5 ml d'HCl 1N dans 50,0 ml d'eau distillée.
- Dissoudre 0,1 g de trypsine cristallisée (SIGMA réf. T8253) dans 10 ml d'HCl 0,05N.
- Aliquoter en fractions de 0,1 ml et conserver à -20°C.
- Préparer la solution C suivante :

| | |
|---|---|
| 1,0 M K₂HPO₄ | 5,0 ml |
| eau distillée QSP | 50 ml |

- Préparer la solution D suivante :

| | |
|---|---|
| 1,0 M KH₂PO₄ | 1,0 ml |
| eau distillée QSP | 10 ml. |

- Ajouter de la solution D à la solution C sous agitation jusqu'à obtention du pH 7,7.
- Aliquoter par 10 ml et conserver à -20°C.
- Préparer 1,0 ml de trypsine 0,1 % par addition de 0,9 ml de tampon phosphate pH 7,7 au 0,1 ml de la solution stock de trypsine 1,0 %.
- Ajouter 1 ml de trypsine 0,1 % à 0,5 ml de culot d'hématies EC₃b ou EC₃ préparées comme décrit en a) dans un bécher de 25 ml, mélanger et incuber pendant 30 minutes à 37°C.
- Laver quatre fois les hématies avec 20 ml de tampon PBS puis éliminer le dernier surnageant de lavage.
- Ajouter 14,5 ml de tampon PBS au culot cellulaire. On obtient ainsi une suspension à 3 % d'hématies présentant le déterminant C₃d du complément à leur surface (ces hématies sont appelées EC₃d).

### 2) Protocole d'immunisation des souris.

Un protocole d'immunisation susceptible de favoriser l'induction d'anticorps à haute affinité a été choisi (c'est-à-dire une immunisation répétitive de très longue durée : jusqu'à 6 mois).

Les souris subissent 2 fois par semaine des injections par voie intrapéritonéale avec 300 µl de la suspension d'hématies EC₃d à 3 % décrite plus haut. La dernière injection est réalisée par voie intraveineuse.

Trois jours après cette dernière injection, la rate des souris est prélevée et homogénéisée ; l'ensemble des cellules est mis à fusionner avec 2.10⁷ cellules de myélome. La sélection des hybridomes est ensuite réalisée, classiquement, en milieu HAT. Le criblage spécifique est effectué 15 jours plus tard.

### 3) Sélection d'hybridomes sécrétant un taux élevé d'anticorps anti-C₃d.

Le criblage des hybridomes s'effectue en microplaques à fond en U, en présence d'hématies recouvertes de la fraction C₃d du complément.

Deux artifices sont utilisés pour favoriser la sélection des clones producteurs d'un taux élevé d'anticorps :

### 3-a- Choix de l'antigène révélateur

Pour détecter les clones producteurs d'anticorps monoclonal anti-C₃d, on utilise comme révélateur des hématies d'un patient particulièrement choisi dans ce but, c'est-à-dire un patient présentant une anémie hémolytique chronique à agglutinines froides, dont on a démontré au laboratoire que les hématies sont couvertes de la fraction C₃d du complément.

### 3-d- Criblage en titrage

Le criblage des hybridomes n'est pas seulement effectué par sélection des clones positifs mais est directement effectué à plusieurs dilutions, de manière à sélectionner les clones produisant l'anticorps au titre le plus élevé.

Après une première sélection, les hybridomes sont reclonés par titrage en dilution limite. Ils sont ensuite amplifiés et retestés pour leur production d'anticorps et pour leur stabilité.

Un clone particulièrement performant a été retenu et un lot pilote et une "banque" en ont été dérivés.

Cette lignée produit un anticorps monoclonal anti-C₃d dont le titre est de 1/512 (dernière dilution donnant une réponse positive).

Contrairement à une notion couramment répandue, le haut titre de cet anticorps n'entraîne pas l'apparition de réponses faussement positives dans les tests de compatibilité sanguine (voir plus loin) mais il améliore très significativement la précision des résultats.

### EXEMPLE IV. Dépistage et identification des anticorps irréguliers dans les sérums des receveurs et des femmes enceintes.

Le procédé de "gel-centrifugation" appliqué au test de Coombs indirect et au test enzymatique à la papaïne a été mis en oeuvre sur 2.050 sérums, avec la gamme d'hématies tests prévues par la loi.
Parmi ces échantillons, 453 présentent des anticorps irréguliers (isolés ou associés). Au total, 615 anticorps irréguliers ont été dépistés et identifiés.

**TABLEAU I**

| SPECIFICITES DES 615 ALLO-ANTICORPS IRREGULIERS DETECTES (COOMBS ET PAPAINE) | | | |
|---|---|---|---|
| ANTICORPS | NOMBRES | ANTICORPS | NOMBRES |
| ANTI-D | 106 | ANTI-Lea | 52 |
| ANTI-C | 72 | ANTI-Leb | 27 |
| ANTI-E | 101 | ANTI-H | 15 |
| ANTI-c | 32 | ANTI-i | 1 |
| ANTI-Cw | 22 | ANTI-U | 1 |
| ANTI-e | 5 | ANTI-GE | 2 |
| ANTI-Ce | 1 | ANTI-LW | 1 |
| ANTI-K | 63 | ANTI-JMH | 1 |
| ANTI-k | 2 | ANTI-Tja | 2 |
| ANTI-Kpa | 5 | ANTI-Vel | 2 |
| ANTI-Fya | 28 | ANTI-Lub | 3 |
| ANTI-Fyb | 1 | ANTI-Wra | 3 |
| ANTI-Jka | 25 | ANTI-Vw | 2 |
| ANTI-Jkb | 4 | ANTI-M | 14 |
| ANTI-S | 5 | ANTI-N | 1 |
| ANTI-s | 1 | ANTI-Pl | 14 |
| | | ANTI-P | 1 |

La sensibilité et la fiabilité du test mis au point ont été comparées à celles des mêmes tests réalisés soit suivant la méthode classique en tube soit en "gel-centrifugation" avec le "kit" vendu par Dia Med.

Dans les tableaux suivants on utilisera pour ces 3 tests les abréviations suivantes : "tube" ; "Dia Med" ; "gel Lille" (pour la présente invention).

Des 615 anticorps irréguliers identifiés au total,
- 1 seul n'est identifié que par la méthode de référence en tube (l'anticorps anti-H)
- 614 sont identifiés en "gel Lille", soit 99,84 %
- 581 sont identifiés par la méthode de référence en tube, soit 94,47 %
- 566 sont identifiés en test "Dia Med", soit 92,03 %.

A une exception près, le procédé de la présente invention est donc plus performant que la méthode de référence et est toujours significativement plus performant que le test "Dia Med".

Une étude détaillée de l'identification des anticorps (portant sur 106 des anticorps détectés précédemment) montre d'importantes discordances entre les 3 procédés. Les résultats détaillés sont présentés en annexe, dans la Figure 1. Les discordances peuvent se résumer comme suit :
- parmi les anti-Rhésus, qui se détectent le mieux dans le test enzymatique à la papaïne, 21 donnent des réactions discordantes sur 339 étudiés, soit 6,2 % :
   4 anti-D, 3 anti-C, 2 anti-e, 1 anti-Cw, 11 anti-E.
- si on se reporte au tableau anti-E de la Figure 1, on voit que

. sur les 19 anti-E détectés en "gel-Lille" (par test à la papaïne),
. 11 ne sont pas détectés en test "Dia Med" (parmi ceux-ci, 3 étaient détectés en tube) et
. 8 ne sont pas détectés en tube.

- parmi les 125 anticorps détectables classiquement par la méthode de Coombs indirecte (anti-Kell, anti-Duffy, anti-Kidd, anti-S, etc.), 19 donnent des réactions discordantes (or il faut noter que les anticorps anti-Kell, Duffy et Kidd, qui peuvent fixer le complément, sont particulièrement dangereux en transfusion) :
   8 anti-Kell, 4 anti-Fya, 5 anti-Jka,
   1 anti-Jkb, 1 anti-S.
- parmi les autres spécificités étudiées, sur 122 anticorps, 19 (soit 15,5 %) donnent des réactions discordantes :
   5 anti-Lea, 3 anti-Leb, 8 anti-M,
   1 anti-P₁, 2 anti-H.
Parmi toutes ces discordances, on constate que le test "Dia Med" ne permet jamais de détecter un anticorps qui n'est pas détecté par une au moins des 2 autres méthodes.

### EXEMPLE V. Utilisation du test pour le groupage ABO-D et le phénotypage des hématies

V.a. Le groupage ABO-D est le phénotypage Rh-Kell sont effectués en présence de sérum AB humain ou d'immuglobulines purifiées, (comme décrit en I), par test enzymatique à la broméline. Dans ce cas particulier on réalise une suspension d'hématies à 1 % (ou d'hématies-tests pour la contre-épreuve du groupe ABO), dans une solution de broméline à 5 ‰ en eau physiologique (ou de l'eau physiologique pour certains anticorps). L'incubation est réalisée à température ambiante pendant 10 minutes.
Les réactifs suivants sont utilisés et incorporés dans le gel :
- anticorps monoclonaux (sélectionnés par la Demanderesse) :
   anti-D, anti-C, anti-E
- anticorps polyclonaux humains contrôlés :
   anti-c, anti-Cw, anti-e.
   V.b. D'autres phénotypages érythrocytaires sont effectués en "gel à l'antiglobuline", (anti-IgG humaine ou antiglobuline polyvalente, standardisée et contrôlée et anti-complément).
   Ce phénotypage concerne plus particulièrement les antigènes Kell, Duffy et S.
   Pour ce test, on utilise une concentration d'hématies de 1 %.

Pour le phénotypage Fya qui est particulièrement délicat, la solution saline d'anticorps est additionnée de 2 g par litre de méthylcellulose.

### V.c. Résultats

Le procédé mis en oeuvre en "gel Lille - centrifugation" est particulièrement performant et ceci apparaît plus particulièrement dans l'étude des doubles populations :
- l'anti-D détecte 4 % d'hématies D⁺ dans 96 % de D⁻ et 6 % de D⁻ dans 94 % de D⁺
- l'anti-C détecte 10 % d'hématies C⁺ dans 90 % de C⁻ et 10 % de C⁻ dans 90 % de C⁺
- l'anti-E détecte 8 % d'hématies E⁺ dans 92 % de E⁻ et 10 % de E⁻ dans 90 % de E⁺
- l'anti-c détecte 6 % d'hématies c⁺ dans 94 % de c⁻
- l'anti-e détecte 8 % d'hématies e⁺ dans 92 % de e⁻ et 10 % de e⁻ dans 90 % de e⁺
- l'anti-Cw détecte 8 % d'hématies Cw⁺ dans 92 % de Cw⁻
- l'anti-Kell détecte 5 % d'hématies K⁺ dans 95 % de K⁻ et 10 % de K⁻ dans 90 % de K⁺
- l'anti-Duffy détecte 10 % d'hématies Fy⁺ dans 90 % de Fy⁻ 10 % de Fy⁻ dans 90 % de Fy⁺
- l'anti-S détecte 10 % d'hématies S⁺ dans 90 % de S⁻.

### EXEMPLE VI. Test de Coombs direct

Le procédé de la présente invention peut également être mis en oeuvre pour effectuer un "test de Coombs direct". Ce test permet de mettre en évidence des anticorps et/ou du complément fixés in vivo sur des hématies de malades. Ce test s'applique particulièrement au diagnostic des anémies hémolytiques auto-immunes (dues à des auto-anticorps anti-hématies dirigés contre des antigènes normalement présents) ou des anémies hémolytiques d'allo-immunisation foeto-maternelle ou post-transfusionnelle.

Dans le cas du test de Coombs direct, on utilise le procédé dans les conditions suivantes :
- On incorpore dans le gel le réactif de Coombs (15 ml pour 100 ml de gel), c'est-à-dire
   - soit de l'antiglobuline polyvalente
   - soit de l'antiglobuline anti-IgG
   - soit de l'anti-complément monoclonal de spécificité anti-C₃d (voir plus haut, exemple III).
- On utilise comme référence négative un gel neutre, sans réactif de Coombs.
- La suspension d'hématies du patient est préparée à 1 % et, comme dans les tests précédents, on en dépose 50 µl à la surface du gel.
- On effectue une centrifugation de 9 minutes à 430 g.

Sur 150 tests effectués, on a observé une sensibilité au moins égale et parfois supérieure à celle de la même technique pratiquée en tube.

### EXEMPLE VII. Diagnostic d'accident hémolytique de transfusion sanguine.

Le procédé de la présente invention peut également être mis en oeuvre pour le diagnostic des accidents transfusionnels dont dépendra la sécurité de transfusions ultérieures.

Ce diagnostic s'effectue après "élution directe", c'est-à-dire que l'anticorps responsable de l'accident est détaché (élué) des hématies incompatibles transfusées par erreur, par un mécanisme physico-chimique. En particulier, la Demanderesse a amélioré le procédé d'élution à pH acide (pH3) décrit par Rekvig et Hannestd (Vox Sang. 33, 280-285, 1977) qui évite l'hémolyse des hématies sensibilisées.

L'élution est réalisée en mettant en contact un volume de culot globulaire et 1,5 volume de tampon HCl-glycine à pH2 de façon à améliorer le rendement de l'élution. Pour éviter l'hémolyse des hématies traitées à pH2, de l'EDTA est ajouté au tampon HCl-glycine.

On facilite ainsi l'identification ultérieure de l'anticorps après neutralisation du pH et recomplémentation de l'éluat.

Les anticorps élués sont beaucoup mieux mis en évidence par le procédé de "gel-centrifugation" que par la technique classique en tube.

En particulier on a mis en évidence les anticorps élués suivants :
anti-D, anti-C, anti-c, anti-K, anti-E, anti-e,
anti-Fya, anti-Jka, anti-Fyb, anti-Jkb, anti-S,
anti-s et anti-Lea.

## Revendications

1. Procédé pour la mise en évidence d'une agglutination érythrocytaire par la rétention sélective des agglutinats après centrifugation en milieu gélifié, en microtubes, adapté aux tests d'immunohématologie classiques, directs ou indirects, caractérisé en ce que
- le milieu gélifié est constitué de 2 gels de finesse de réticulation différente,
- le milieu gélifié contient un ou plusieurs réactifs spécifiquement adaptés à chaque type de test,
- les conditions de mise en oeuvre de chaque test sont particulièrement adaptées à ce procédé de centrifugation en milieu gélifié.

2. Procédé selon la revendication 1, caractérisé en ce que le milieu gélifié est constitué d'un mélange d'un dextran dont les particules ont un diamètre de 20 à 50 µm et d'un autre dextran dont les particules ont un diamètre de 10 à 23 µm.

3. Procédé selon la revendication 1 ou 2, caractérisé en ce que le milieu gélifié contient un sérum AB humain.

4. Procédé selon la revendication 1 ou 2, caractérisé en ce que le milieu gélifié contient des antiglobulines.

5. Procédé selon l'une quelconque des revendications 1, 2 ou 4, caractérisé en ce que le milieu gélifié contient de l'anticorps anticomplément monoclonal.

6. Procédé selon la revendication 5, caractérisé en ce que l'anticorps monoclonal est un anticorps de spécificité anti-C₃d et de haute affinité.

7. Procédé selon la revendication 5 ou 6, caractérisé en ce que l'anticorps anti-C₃d est produit par un hybridome induit et sélectionné en vue dudit procédé.

8. Procédé selon la revendication 7, caractérisé en ce que la sélection de l'hybridome est effectuée en titrage pour obtenir un hybridome producteur d'anticorps monoclonal de très haut titre.

9. Procédé selon l'une quelconque des revendications 1, 2, 4, 5, 6, caractérisé en ce que la mise en oeuvre du test comprend l'addition d'EDTA disodique dans la suspension d'hématies.

10. Procédé selon la revendication 9, caractérisé en ce que la concentration d'EDTA disodique est comprise entre 0,15 et 0,30%.

## Patentansprüche

1. Verfahren zum Nachweis einer Erythrocytenagglutination durch selektive Retention der Agglutinate nach Zentrifugation in Gelmedium, in Mikroröhrchen, welches an herkömmliche direkte oder indirekte Immunhämatologietests angepaßt ist, **dadurch gekennzeichnet,** daß
das Gelmedium aus zwei Gelen mit unterschiedlich feiner Vernetzung besteht,
das Gelmedium ein oder mehrere Reagenzien enthält, die spezifisch an jede Art von Test angepaßt sind,
die Bedingungen für die Ausführung eines jedes Tests speziell an das Verfahren der Zentrifugation in Gelmedium angepaßt sind.

2. Verfahren nach Anspruch 1, **dadurch gekennzeichnet,** daß das Gelmedium aus einem Gemisch aus einem Dextran, dessen Teilchen einen Durchmesser von 20 bis 50 µm haben, und aus einem weiteren Dextran, dessen Teilchen einen Durchmesser von 10 bis 23 µm haben, besteht.

3. Verfahren nach Anspruch 1 oder 2, **dadurch gekennzeichnet,** daß das Gelmedium ein menschliches AB-Serum enthält.

4. Verfahren nach Anspruch 1 oder 2, **dadurch gekennzeichnet,** daß das Gelmedium Antiglobuline enthält.

5. Verfahren nach einem der Ansprüche 1, 2 oder 4, **dadurch gekennzeichnet,** daß das Gelmedium einen monoklonalen Anti-Komplement-Antikörper enthält.

6. Verfahren nach Anspruch 5, **dadurch gekennzeichnet,** daß der monoklonale Antikörper ein Antikörper mit Anti-C₃d-Spezifität und hoher Affinität ist.

7. Verfahren nach Anspruch 5 oder 6, **dadurch gekennzeichnet,** daß der Anti-C₃d-Antikörper von einem Hybridom produziert wird, das im Hinblick auf das genannte Verfahren induziert und selektiert wurde.

8. Verfahren nach Anspruch 7, **dadurch gekennzeichnet,** daß die Selektion des Hybridoms dadurch Titration ausgeführt wird, um ein hochtitrigen monoklonalen Antikörper-produzierendes Hybridom zu erhalten.

9. Verfahren nach einem der Ansprüche 1, 2, 4, 5, 6, **dadurch gekennzeichnet,** daß die Ausfühhrung des Tests die Zugabe von Dinatrium-EDTA in die Erythrocytensuspension umfaßt.

10. Verfahren nach Anspruch 9, **dadurch gekennzeichnet,** daß die Dinatrium-EDTA-Konzentration zwischen 0,15 und 0,30% liegt.

## Claims

1. Process for demonstrating erythrocyte agglutination by the selective retention of the agglutinated products after centrifugation in a gelled medium, in microtubes, adapted to direct or indirect conventional immunohaematology tests, characterised in that
- the gelled medium comprises 2 gels having different thinness of reticulation,
- the gelled medium contains one or more reactants specifically adapted to each type of test,
- the conditions for carrying out each test are specially adapted to that method of centrifugation in gelled medium.

2. Process according to claim 1, characterised in that the gelled medium comprises a mixture of one dextran of which the particles have a diameter of from 20 to 50 µm and another dextran of which the particles have a diameter of from 10 to 23 µm.

3. Process according to claim 1 or claim 2, characterised in that the gelled medium contains a human AB serum.

4. Process according to claim 1 or claim 2, characterised in that the gelled medium contains antiglobulins.

5. Process according to any one of claims 1, 2 and 4, characterised in that the gelled medium contains a monoclonal anticomplement antibody.

6. Process according to claim 5, characterised in that the monoclonal antibody is an antibody having anti-C₃d specificity and high affinity.

7. Process according to claim 5 or claim 6, characterised in that the anti-C₃d antibody is produced by a hybridoma induced and selected with a view to the said process.

8. Process according to claim 7, characterised in that the selection of the hybridoma is carried out by titration in order to obtain a hybridoma that produces high titer monoclonal antibodies.

9. Process according to any one of claims 1, 2, 4, 5 and 6, characterised in that carrying out the test comprises the addition of disodium EDTA to the suspension of erythrocytes.

10. Process according to claim 9, characterised in that the concentration of disodium EDTA is from 0.15 to 0.30 %.
